# EUROPÄISCHE PATENTSCHRIFT

(11) **EP 3 962 362 B1**
(45) Veröffentlichungstag und Bekanntmachung des Hinweises auf die Patenterteilung: **07.06.2023**
(21) Anmeldenummer: 20723381.8
(22) Anmeldetag: 30.04.2020
(51) Int. Cl.: A61B 5/1455, G01N 21/25, G01N 21/31

(54) **OPTISCHER SENSOR ENTHALTEND EINEN WELLENLEITER MIT HOLOGRAPHISCHEN ELEMENTEN ZUR MESSUNG DES PULSES UND DER BLUTSAUERSTOFFSÄTTIGUNG**
OPTICAL SENSOR COMPRISING A WAVGUIDE WITH HOLOGRAPHIC ELEMENTS FOR MEASURING PULSE AND BLOOD OXYGEN SATURATION
CAPTEUR OPTIQUE COMPRENANT UN GUIDE D'ONDE AVEC DES ELEMENTS HOLOGRAPHIQUES POUR MESURER L'IMPULSION ET LA SATURATION EN OXYGENE DU SANG

(30) Priorität: 03.05.2019 DE 102019206361
(43) Veröffentlichungstag der Anmeldung: 09.03.2022
(73) Patentinhaber: Audi AG, 85045 Ingolstadt (DE)
(72) Erfinder: KLUG, Markus, 85057 Ingolstadt (DE); MOLL, Tobias, 85055 Ingolstadt (DE); SCHEUCHENPFLUG, Johannes, 85107 Baar-Ebenhausen (DE)
(74) Vertreter: Hofstetter, Schurack & Partner
(86) Internationale Anmeldenummer: PCT/EP2020/062091
(87) Internationale Veröffentlichungsnummer: WO 2020/225111

(56) Entgegenhaltungen:
- US-A- 5 088 817
- US-A1- 2012 310 060
- US-A1- 2013 274 611
- US-B2- 10 146 053
- Jingjing Guo ET AL: "Design of a multiplexing grating for color holographic waveguide", Optical Engineering, vol. 54, no. 12, 22 December 2015 (2015-12-22), page 125105, XP055767890, BELLINGHAM ISSN: 0091-3286, DOI: 10.1117/1.OE.54.12.125105

## Beschreibung

Die Erfindung betrifft eine Spektrometrievorrichtung zur nichtinvasiven Messung von zumindest einem medizinischen Kennwert an einem biologischen Gewebe.

Um einen medizinischen Kennwert von einem biologischen Gewebe, beispielsweise eines Gewebes eines menschlichen oder tierischen Körpers, zu bestimmen, ist es oftmals notwendig, eine Gewebeprobe zu entnehmen und diese zu analysieren. Dabei kann das biologische Gewebe Epithelgewebe, Binde- und Stützgewebe, Muskelgewebe und Nervengewebe umfassen. Außerdem kann das biologische Gewebe auch Gewerbeflüssigkeit, insbesondere Blut, umfassen oder der medizinische Kennwert kann beispielsweise ein Pulsschlag und/oder eine Sauerstoffsättigung des Blutes sein.

Die Bestimmung des Pulsschlags und/oder der Sauerstoffsättigung des Blutes können aber auch nichtinvasiv, also ohne eine Entnahme von Gewebe, bestimmt werden. Hierbei ist bekannt, mittels einer Spektrometrievorrichtung, insbesondere mit einem Pulsoximeter, eine Pulsfrequenz und eine Sauerstoffsättigung des Blutes nichtinvasiv zu bestimmen. Dabei wird für die Pulsfrequenzbestimmung Licht mit einer vorbestimmten Wellenlänge in das biologische Gewebe abgestrahlt, wobei das Licht, das durch das Gewebe hindurchgegangen ist beziehungsweise von dem Gewebe reflektiert wurde, anschließend mittels eines Detektors gemessen werden kann. Aus einer Änderung des so gemessenen Signals kann dann die Pulsfrequenz bestimmt werden. Um die Sauerstoffsättigung des Blutes zu bestimmen, kann zusätzlich Licht mit einer anderen vorbestimmten Wellenlänge in das Gewebe abgestrahlt und gemessen werden, damit mit bekannten Methoden die Sauerstoffsättigung des Blutes ermittelt werden kann. Die Messung bezieht sich insbesondere auf die Strahlungsintensität des Lichts. US 2013/274611 A1 beschreibt eine derartige Spektrometrievorrichtung und offenbart gleichzeitig alle Merkmale des Oberbegriffs von Anspruch 1.

Bei bekannten Spektrometrievorrichtungen zur nichtinvasiven Messung von einem medizinischen Kennwert an einem biologischen Gewebe, wie beispielsweise einem Pulsoximeter, ist es messartbedingt vorgesehen, dass die Lichtquelle und der Detektor nahe am Messort verbaut werden müssen, wodurch sich eine Einschränkung in der Bauweise dieser Spektrometrievorrichtungen ergibt.

Der Erfindung liegt die Aufgabe zugrunde, zur nichtinvasiven Messung von zumindest einem medizinischen Kennwert an einem biologischen Gewebe eine Spektrometrievorrichtung bereitzustellen, deren messartbedingter Nachteil des geringen Abstands in alternativer Weise überwunden werden kann.

Die Aufgabe wird durch den Gegenstand des unabhängigen Patentanspruchs gelöst. Vorteilhafte Weiterbildungen der Erfindung sind durch die abhängigen Patentansprüche, die folgende Beschreibung sowie die Figuren offenbart.

Durch die Erfindung ist eine Spektrometrievorrichtung zur nichtinvasiven Messung an einem biologischen Gewebe bereitgestellt, um an dem biologischen Gewebe zumindest einen medizinischen Kennwert zu messen. Nichtinvasiv meint hier insbesondere, dass kein mechanischer Eingriff in das Gewebe erfolgt. Die Spektrometrievorrichtung umfasst ein Trägermedium, welches ausgebildet ist, als Lichtleiter eingekoppeltes Licht mittels interner Reflexion zu übertragen oder zu leiten, wobei an dem Trägermedium ein Transceiverbereich und ein Messbereich, der entlang einer Längserstreckungsrichtung des Trägermediums bezüglich des Transceiverbereichs versetzt angeordnet und zum Auflegen des biologischen Gewebes vorgesehen ist, bereitgestellt ist. Mit anderen Worten liegt ein Trägermedium vor, welches Licht z.B. in einem transparenten Feststoff mittels Totalreflexion leiten kann, und das einen Transceiverbereich und einen Messbereich aufweist, welche beabstandet zueinander angeordnet sind, wobei auf dem Messbereich biologisches Gewebe aufgelegt werden kann. Mittels des Trägermediums kann somit Licht zwischen dem Transceiverbereich und dem Messbereich hin und her übertragen werden, wodurch eine Beabstandung einer Lichtquelle und einer Detektionsvorrichtung von einem zu vermessenden Gewebe erreicht werden kann. Das biologische Gewebe kann insbesondere ein Finger einer Person sein , dessen medizinischer Kennwert gemessen werden soll. Gemäß der Erfindung kann der medizinische Kennwert vorzugsweise eine Pulsfrequenz und zusätzlich eine Blutsauerstoffsättigung sein. Das Trägermedium kann vorzugsweise einen Lichtwellenleiter aus Glas oder Kunststoff oder einer Kombination von mehreren lichtleitenden Materialien umfassen. Es kann auf einer Platte oder einem Stab oder mehreren Platten in Sandwichbauweise oder einer Mehrschichtanordnung aus Folien oder Lacken beruhen.

Diese Spektrometrievorrichtung umfasst ferner eine Transceivervorrichtung mit zumindest einer ersten Lichtquelle und einer Detektionsvorrichtung, wobei die erste Lichtquelle dazu ausgebildet und angeordnet ist, Licht mit einer ersten Wellenlänge auf den Transceiverbereich abzustrahlen. Der Transceiverbereich ist mit einer vorgegebenen Transceiverablenkstruktur ausgebildet, die dazu ausgelegt ist, zumindest das von der ersten Lichtquelle abgestrahlte Licht der ersten Wellenlänge in das Trägermedium in Richtung des Messbereichs einzukoppeln. Das bedeutet, dass eine erste Lichtquelle der Transceivervorrichtung Licht mit einer ersten Wellenlänge auf den Transceiverbereich des Trägermediums abstrahlt und eine vorgegebene Transceiverablenkstruktur dieses Licht in das Trägermedium einkoppelt, damit das eingekoppelte Licht zu dem beabstandeten Messbereich umgelenkt und dorthin weitergeleitet werden kann. Vorzugsweise kann dabei die erste Lichtquelle als eine Leuchtdiode oder eine Laserdiode ausgebildet sein, die Licht der vorgegebenen Wellenlänge abstrahlen kann.

Der besagte Messbereich ist mit einer vorgegebenen Messablenkstruktur ausgebildet, die dazu ausgelegt ist, zumindest in das Trägermedium in Richtung des Messbereichs eingekoppeltes Licht, insbesondere das Licht der ersten Wellenlänge, aus dem Trägermedium auszukoppeln, sodass bei aufgelegtem biologischen Gewebe auf eine Messoberfläche des Trägermediums im Messbereich das biologische Gewebe das ausgekoppelte Licht außerhalb des Trägermediums reflektiert, und wobei die vorgegebene Messablenkstruktur ferner dazu ausgebildet ist, das von außerhalb des Trägermediums reflektierte Licht zu empfangen und derart abzulenken oder einzukoppeln oder zu beugen, dass es zurück in das Trägermedium in Richtung des Auskoppelbereichs eingekoppelt wird. Die vorgegebene Transceiverablenkstruktur des Transceiverbereichs ist ferner dazu ausgebildet, das Licht, das von dem Messbereich zurück in das Trägermedium in Richtung des Auskoppelbereichs eingekoppelt wird, aus dem Trägermedium auf die Detektionsvorrichtung der Transceivervorrichtung auszukoppeln, wobei die Detektionsvorrichtung dazu ausgebildet ist, ein erstes Intensitätssignal der ersten Wellenlänge zu bestimmen. Ferner umfasst die Spektrometrievorrichtung eine Auswerteeinrichtung, die dazu ausgebildet ist, mittels eines zeitlichen Verlaufs des ersten Intensitätssignals ein Pulsfrequenzsignal und/oder Pulsverlaufssignal als medizinischen Kennwert zu bestimmen.

Mit anderen Worten wird Licht mit einer vorgegebenen Wellenlänge von einer ersten Lichtquelle der Transceivervorrichtung auf den Transceiverbereich des Trägermediums abgestrahlt, wobei der Transceiverbereich eine vorgegebene Transceiverablenkstruktur aufweist, die das Licht in das Trägermedium einkoppelt, wo es durch interne Reflexion durch das Trägermedium zu dem beabstandeten Messbereich weitergeleitet wird, an dem die Messablenkstruktur das in das Trägermedium eingekoppelte Licht aus dem Trägermedium auskoppelt und auf ein aufgelegtes biologisches Gewebe, insbesondere einen Finger einer Person, strahlen kann. An dem aufgelegten biologischen Gewebe kann dann das Licht reflektiert werden und zurück über die vorgegebene Messablenkstruktur des Messbereichs zurück in das Trägermedium eingekoppelt werden. Das zurück in das Trägermedium eingekoppelte Licht kann dann mittels interner Reflexion innerhalb des Trägermediums zurück zu der vorgegebenen Transceiverablenkstruktur des Transceiverbereichs geleitet werden, wo es dann auf die Detektionsvorrichtung der Transceivervorrichtung auskoppelt werden, die ein Intensitätssignal der ersten Wellenlänge bestimmen kann. Eine Auswerteeinrichtung kann anschließend mittels einer vorgegebenen Bestimmungsmethode ein Pulsfrequenzsignal und/oder ein Pulsverlaufsignal als medizinischen Kennwert bestimmen. Eine geeignete Bestimmungsmethode kann der Fachmann dem Stand der Technik entnehmen, wie eingangs beschrieben wurde.

Die vorgegebene Transceiverablenkstruktur und die vorgegebene Messablenkstruktur, die zumindest ein optisches Gitter aufweisen, können als Beugungsstruktur oder Brechungsstruktur, als eine Interferenzstruktur, Gitterstruktur, als Linsensystem oder Spiegel ausgebildet sein. Insbesondere können die Transceiverablenkstruktur und die Messablenkstruktur jeweils als holographisches optisches Element (HOE) (oder abgekürzt holographisches Element) ausgebildet sein, das Licht mit einer vorgegebenen Wellenlänge in einen vorgegebenen Winkel ablenken kann. Die Detektionsvorrichtung kann vorzugsweise als Fotodiode, beispielsweise als CCD-Sensor oder CMOS-Sensor, ausgebildet sein, der eine Lichtintensität beziehungsweise einen Intensitätsverlauf des Lichts, bestimmen kann. Die Auswerteeinrichtung, die als Computerprozessor ausgebildet sein kann, kann anschließend in dem Intensitätssignal ein periodisches Muster erkennen, das dann in ein Pulsfrequenzsignal umgewandelt werden kann und zusätzlich oder alternativ als Pulsverlaufsignal angezeigt oder ausgegeben werden kann.

Durch die Erfindung ergibt sich der Vorteil, dass der Messbereich beabstandet von dem Transceiverbereich angeordnet werden kann, wodurch eine flachere Bauweise des Messbereichs erreicht werden kann. Hierdurch ist es beispielsweise möglich, den Messbereich als Messort des biologischen Gewebes in eine flache Struktur anzuordnen und somit einem Benutzer einen einfacheren Zugang zu dem Messbereich zu ermöglichen. Auch eine Reinigung der Auflagefläche des Fingers im Messbereich ist durch die beabstandete Anordnung einfacher, wodurch die Hygiene verbessert werden kann. Zu der Erfindung gehören auch Ausführungsformen, durch die sich zusätzliche Vorteile ergeben.

Vorzugsweise ist vorgesehen, dass die erste Wellenlänge in einem Bereich von 600 Nanometern bis 800 Nanometern liegt, insbesondere bei 660 Nanometern. Hierbei ist dem Fachmann bekannt, dass eine Wellenlängenangabe nicht ausschließlich eine diskrete Wellenlänge angibt, sondern dass die Wellenlänge eine Optimalwellenlänge oder Spitzenwellenlänge ("peak wavelength") angibt, die um einige Nanometer schwanken kann. Beispielsweise kann bei der Wellenlänge von 660 Nanometern auch eine Wellenlänge von +/- 5 Prozent der Wellenlänge gemeint. Der hier angegebene erste Wellenlängenbereich hat den Vorteil, dass eine Lichtabsorption des biologischen Gewebes, insbesondere des menschlichen Blutes, eine bevorzugte Absorptions- beziehungsweise Reflexionscharakteristik aufweist.

Die Transceivervorrichtung umfasst ferner eine zweite Lichtquelle, die dazu ausgebildet und angeordnet ist, Licht mit einer zweiten Wellenlänge auf den Transceiverbereich abzustrahlen, wobei die vorgegebene Transceiverablenkstruktur des Transceiverbereichs ferner dazu ausgebildet ist, das von der zweiten Lichtquelle abgestrahlte Licht der zweiten Wellenlänge in das Trägermedium in Richtung des Messbereichs einzukoppeln. Die Detektionsvorrichtung ist ferner dazu ausgebildet, ein zweites Intensitätssignal der zweiten Wellenlänge zu bestimmen, wobei die Auswerteeinrichtung ferner dazu ausgebildet ist, aus dem ersten Intensitätssignal und dem zweiten Intensitätssignal eine Blutsauerstoffsättigung als medizinischen Kennwert zu bestimmen.

Mit anderen Worten ist in der Transceivervorrichtung eine zweite Lichtquelle vorgesehen, die Licht mit einer zweiten Wellenlänge auf den Transceiverbereich abstrahlt, dessen Transceiverablenkstruktur dann das Licht mit der zweiten Wellenlänge in das Trägermedium einkoppeln kann. Die erste und die zweite Wellenlänge unterscheiden sich. In dem Trägermedium wird das Licht mit der zweiten Wellenlänge dann zu dem Messbereich übertragen, wobei das übertragene Licht an der Messablenkstruktur auf ein im Messbereich befindliches biologisches Gewebe abgelenkt werden kann. An dem biologischen Gewebe kann das Licht dann reflektiert werden und zurück über die Messablenkstruktur in das Trägermedium eingekoppelt werden, wo es dann den Weg zu der Transceiverablenkstruktur zurücklegen kann.

Die Transceiverablenkstruktur kann dann das Licht der zweiten Wellenlänge zu der Detektionsvorrichtung auskoppeln, die dann ein zweites Intensitätssignal des Lichts der zweiten Wellenlänge bestimmen kann. Aus dem Intensitätssignal der ersten Wellenlänge und dem Intensitätssignal der zweiten Wellenlänge bestimmt die Auswerteeinrichtung mittels einer bekannten Methode eine Blutsauerstoffsättigung als medizinischen Kennwert.

Eine solche Methode wurde eingangs bereits als im Stand der Technik verfügbar beschrieben. Die zweite Lichtquelle kann als Leuchtdiode oder Laserdiode ausgebildet sein und die Transceiverablenkstruktur sowie die Messablenkstruktur sind derart ausgebildet, dass sie Licht mit der ersten und zweiten Wellenlänge in das Trägermedium ein- und auskoppeln können. Hierfür ist zumindest ein optisches Gitter vorgesehen und beispielsweise eine Spiegelanordnung, eine Linsenanordnung, eine Beugungsstruktur oder eine Brechungsstruktur, insbesondere ein holographisch-optisches Element (HOE). Durch diese Ausführungsform ergibt sich der Vorteil, dass zusätzlich zu einem Pulsfrequenzsignal eine Blutsauerstoffsättigung bestimmt werden kann.

Vorzugsweise ist vorgesehen, dass die zweite Wellenlänge in einem Bereich von 850 Nanometern bis 1000 Nanometern liegt, insbesondere bei 940 Nanometern. Dies ist vorteilhaft, da eine Absorptions- beziehungsweise Reflexionscharakteristik von sauerstoffarmem zu sauerstoffreichem Blut in diesem Wellenlängenbereich eine andere Charakteristik aufweist als bei der ersten Wellenlänge. Somit kann eine Konzentration von sauerstoffreichem Blut genauer bestimmt werden.

Eine Ausführungsform sieht vor, dass die Detektionsvorrichtung dazu ausgebildet ist, zwischen der ersten Wellenlänge und der zweiten Wellenlänge zu unterscheiden. Dies kann beispielsweise dadurch erreicht werden, dass die erste Lichtquelle und die zweite Lichtquelle ein alternierendes Signal ausstrahlen und die Detektionsvorrichtung das Intensitätssignal je nach abstrahlender Lichtquelle zuordnen kann. Es kann jedoch auch vorgesehen sein, dass die Detektionsvorrichtung einen Strahlteiler aufweist, der auf die Detektionsvorrichtung eintreffendes Licht aufteilt und je nach Wellenlänge auf unterschiedliche Fotodioden leiten kann. Alternativ oder zusätzlich können Farbfilter, die beispielsweise über zumindest zwei Fotosensoren angeordnet werden, verwendet werden. Durch diese Ausführungsform ergibt sich der Vorteil, dass eine verbesserte Intensitätsunterscheidung der unterschiedlichen Wellenlängen erreicht werden kann, wodurch eine genauere Bestimmung des medizinischen Kennwerts erreicht werden kann.

Eine weitere Ausführungsform sieht vor, dass der Transceiverbereich und die Transceivervorrichtung in einem vom Außenlicht geschützten Gehäuse untergebracht sind. Mit anderen Worten sind der Transceiverbereich und die Transceivervorrichtung in einem lichtundurchlässigen Gehäuse untergebracht und nur das Trägermedium führt aus dem Gehäuse hinaus zu dem Messbereich, der außerhalb des Gehäuses liegt. Das bedeutet, dass nur Licht, das in das Trägermedium eingekoppelt wird, zu der Transceivervorrichtung geleitet wird. Durch diese Ausführungsform ergibt sich der Vorteil, dass ein Einfluss des Umgebungslichts auf die die Transceivervorrichtung, insbesondere die Detektionsvorrichtung, minimiert werden kann und somit eine Abschwächung eines Hintergrundrauschens in der Detektionsvorrichtung erreicht werden kann, wodurch sich die Bestimmung des medizinischen Kennwerts verbessert.

Die Erfindung sieht vor, dass die vorgegebene Transceiverablenkstruktur und die vorgegebene Messablenkstruktur zumindest ein optisches Gitter aufweisen.

Optische Gitter, auch Beugungsgitter genannt, sowie deren Wirkungsweise und Herstellungsverfahren sind dabei allgemein bekannt. Grundsätzlich können optische Gitter als zumindest abschnittsweise periodische Strukturen, sogenannte Gitterstrukturen, in einem Substrat ausgebildet sein, die durch den physikalischen Effekt der Beugung eine Lichtlenkung, wie sie zum Beispiel von Spiegeln, Linsen oder Prismen bekannt ist, herbeiführen können. Fällt Licht, d.h. fallen Lichtstrahlen auf das optische Gitter, wobei die einfallenden Lichtstrahlen insbesondere die Bragg-Gleichung erfüllen, werden die Lichtstrahlen durch das optische Gitter gebeugt oder abgelenkt. Die Lichtlenkung kann somit insbesondere durch Interferenzerscheinungen der durch das optische Gitter gebeugten Lichtstrahlen erfolgen. Die Ablenkstruktur kann entsprechend auch als Beugungsstruktur bezeichnet werden. Ein holographisches Oberflächengitter und ein holographisches Volumengitter sind holographisch-optische Elemente, die insbesondere durch ein Holographieverfahren hergestellt werden können.

Vorzugsweise kann ein optisches Gitter gegenüber dem einfallenden Licht winkel- beziehungsweise richtungsselektiv und/oder wellenlängen- beziehungsweise frequenzselektiv ausgebildet sein. Somit kann nur Licht, das aus einer vorbestimmten Einfallsrichtung, zum Beispiel in einem vorbestimmten Winkel, auf ein optisches Gitter fällt, abgelenkt werden. Licht, das aus einer anderen Richtung auf das optische Gitter fällt, wird vorzugsweise nicht abgelenkt oder umso weniger, je größer der Unterschied zur vorbestimmten Einfallsrichtung ist. Nicht unter den Umfang der Ansprüche fällt die Ausführungsform, in der zusätzlich oder alternativ nur Licht einer Wellenlänge oder Licht, welches höchstens um einen vorbestimmten Wellenlängenbereich von der vorbestimmten Wellenlänge abweicht, von dem optischen Gitter in einem bestimmten Beugungswinkel abgelenkt wird.

Anders formuliert kann beispielsweise eine Optimalwellenlänge vorgegeben sein, bei der nur ein Anteil des Lichts in einem bestimmten Wellenlängen- oder Frequenzbereich um die Optimalwellenlänge von dem optischen Gitter abgelenkt wird (beispielsweise eine zentrale Optimalwellenlänge und ein Bereich mit Wellenlängenwerten bis +/- 10 Prozent der Optimalwellenlänge), der übrige Anteil des Lichts kann hingegen ohne abgelenkt zu werden durch das Gitter propagieren. Von polychromatischem Licht, welches auf das optische Gitter trifft, kann somit wenigstens ein monochromatischer Lichtanteil abgespaltet werden. Der Ablenkeffekt ergibt sich somit frequenzselektiv und/oder winkelselektiv, wobei der Ablenkeffekt für ein Optimalwellenlänge maximal ist und zu längeren und kürzeren Wellenlängen hin abfällt oder schwächer wird, beispielsweise gemäß einer Gaußglocke abfällt. Insbesondere wirkt der Ablenkeffekt nur auf einen Bruchteil des sichtbaren Lichtspektrums und/oder in einem Winkelbereich kleiner als 90 Grad.

Die Erfindung sieht vor, dass das zumindest eine optische Gitter ein holographisches Oberflächengitter oder ein holographisches Volumengitter ist, das eine Multiplex-Beugungsstruktur für zumindest die erste und die zweite Wellenlänge aufweist. Besonders bevorzugt können optische Gitter mittels Belichtung eines Substrats, also beispielsweise fotolithografisch oder holografisch, hergestellt werden. In diesem Zusammenhang können die optischen Gitter dann auch als holografische oder holografisch-optische Gitter bezeichnet werden. Es sind zwei Arten von holografisch-optischen Gittern bekannt: holografische Oberflächengitter (surface holografic gratings, kurz: SHG) und holografische Volumengitter (volume holografic gratings, kurz: VHG). Bei holografischen Oberflächengittern kann die Gitterstruktur durch optisches Verformen einer Oberflächenstruktur des Substrats erzeugt werden. Durch die veränderte Oberflächenstruktur kann auftreffendes Licht abgelenkt, zum Beispiel reflektiert werden. Beispiele für holografische Oberflächengitter sind sogenannte Sägezahn- oder Blazegitter. Im Gegensatz dazu kann die Gitterstruktur bei holografischen Volumengittern in das ganze Volumen oder einen Teilbereich des Volumens des Substrats eingearbeitet sein. Holografische Oberflächengitter und holografische Volumengitter sind in der Regel frequenzselektiv. Es sind jedoch auch optische Gitter bekannt, die polychromatisches Licht beugen können. Diese werden als holografische Mehrvolumenfachgitter (multiplexed volume holografic gratings, kurz: MVHG) bezeichnet und können beispielsweise durch Verändern der Periodizität der Gitterstruktur eines optischen Gitters oder durch Anordnen mehrerer holografisches Volumengitter hintereinander hergestellt werden, wodurch eine Multiplex-Beugungsstruktur entsteht.

Als Material für ein Substrat zum Einarbeiten eines optischen Gitters eignet sich zum Beispiel besonders Glas, vorzugsweise Quarzglas. Alternativ oder zusätzlich kann auch ein Polymer, insbesondere Fotopolymer, oder eine Folie, insbesondere eine fotosensitive Folie, zum Beispiel aus Kunststoff oder einem organischen Stoff. Zur Verwendung derartiger Substrate, sollte zusätzlich beachtet werden, dass das Material, insbesondere in Substratform, flexible und lichtwellenleitende Eigenschaften aufweist. Substrate die eine Ablenkstruktur zum Beugen von Licht, beispielsweise in Form eines optischen Gitters aufweisen, können auch als holografisch-optische Elemente (HOE) bezeichnet werden.

Eine weitere Ausführungsform sieht vor, dass der Messbereich und der Transceiverbereich direkt in das Trägermedium, insbesondere in eine Oberflächenstruktur des Trägermediums, eingearbeitet sind. Eine alternative Ausführungsform sieht vor, dass das Trägermedium als separates Element zu dem Messbereich und dem Transceiverbereich ausgebildet ist. Im ersten Fall können der Messbereich und der Transceiverbereich somit beispielsweise direkt in eine Oberflächenstruktur des Trägermediums eingearbeitet werden. Somit kann das Trägermedium selbst als HOE ausgebildet sein, beispielsweise geätzt oder gelasert sein. Im zweiten Fall können der Messbereich, der Transceiverbereich und Trägermedium separat ausgebildet sein. Dabei können der Messbereich und der Transceiverbereich beispielsweise wenigstens ein erstes Element bilden und das Trägermedium kann ein zweites Element bilden, welches an dem ersten Element anliegt. Somit können der Messbereich und der Transceiverbereich in wenigstens einem HOE ausgebildet sein. Die ermöglicht eine größere Auswahl bei der Nutzung eines Trägermediums. Beispielsweise können der Messbereich und der Transceiverbereich in unterschiedlichen Abschnitten einer holografischen Folie oder Platte ausgebildet sein. Zum Befestigen der Folie oder Platte an dem Trägermedium kann die Folie oder Platte an das Trägermedium angeklebt sein. Alternativ kann die holografische Folie auch als Adhäsionsfolie ausgebildet sein und direkt, also ohne Klebstoff, durch molekulare Kräfte an der Oberfläche des Trägermediums haften.

Die Erfindung umfasst auch die Kombinationen der Merkmale der beschriebenen Ausführungsformen, soweit sie sich nicht explizit als gegenseitige Alternativen bezeichnet wurden.

Im Folgenden sind Ausführungsbeispiele der Erfindung beschrieben. Hierzu zeigt:
- Fig. 1: eine schematische Darstellung einer Spektrometrievorrichtung, die nicht vom Umfang der Ansprüche erfasst wird;

- Fig. 2: eine schematische Darstellung einer Spektrometrievorrichtung nach einer beispielhaften Ausführungsform

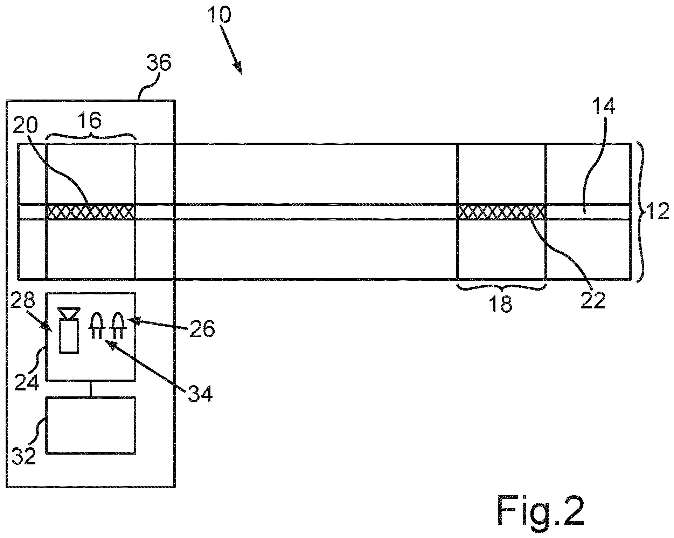

Bei dem in Figur 2 erläuterten Ausführungsbeispiel handelt es sich um eine bevorzugte Ausführungsform der Erfindung. Bei dem Ausführungsbeispiel stellen die beschriebenen Komponenten der Ausführungsform jeweils einzelne, unabhängig voneinander zu betrachtende Merkmale der Erfindung dar, welche die Erfindung jeweils auch unabhängig voneinander weiterbilden. Daher soll die Offenbarung auch andere als die dargestellten Kombinationen der Merkmale der Ausführungsform umfassen. Des Weiteren ist die beschriebene Ausführungsform auch durch weitere der bereits beschriebenen Merkmale der Erfindung ergänzbar.

In den Figuren bezeichnen gleiche Bezugszeichen jeweils funktionsgleiche Elemente.

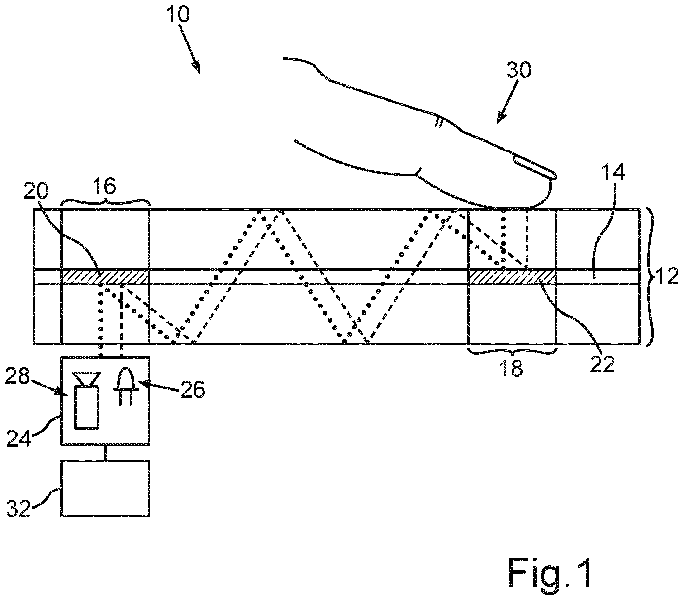

In Fig. 1 ist eine schematische Darstellung einer Spektrometrievorrichtung 10 zur nichtinvasiven Messung von zumindest einem medizinischen Kennwert an einem biologischen Gewebe dargestellt, die nicht unter den Umfang der Ansprüche fällt. Die Spektrometrievorrichtung 10 umfasst ein Trägermedium 12, welches als Lichtleiter zum Übertragen von eingekoppeltem Licht durch interne Reflexion ausgebildet ist.

Das Trägermedium 12 ist hierbei mit separaten quaderförmigen Elementen, also Platten, ausgebildet, die zum Bilden des Trägermediums in einer Sandwichbauweise aufgebaut sind. Hierbei umfasst das Trägermedium 12 beispielsweise zwei Kunststoffplatten oder Glasplatten, die als Lichtleiter dienen und die Deckschichten des Trägermediums bilden. Ein Kern des Trägermediums kann beispielsweise mittels eines holographisch-optischen Elements, das hier als holographisches Element 14 bezeichnet wird, ausgebildet sein, welches beispielsweise als transparente Fotopolymerfolie ausgebildet sein kann. Die Glasplatten liegen mit einer jeweiligen Oberfläche direkt an jeweils gegenüberliegenden Oberflächen des holographischen Elements an. Anders ausgedrückt, liegen das holographisches Element 14 und die Glasplatten flächig mit ihren jeweiligen von einer Längs- und Breitseite eingeschlossenen Flächen aneinander an. Fig. 1 zeigt insbesondere ein Schnittbild der Spektrometrievorrichtung 10, bei dem die Spektrometrievorrichtung 10 mit einem Schnitt entlang einer Längsachse dargestellt ist.

Das Trägermedium 12 kann einen Transceiverbereich 16 und einen Messbereich 18 umfassen, die entlang einer Längserstreckungsrichtung des Trägermediums versetzt angeordnet sind. Insbesondere können der Transceiverbereich 16 und der Messbereich 18, wie in dieser Ausführungsform dargestellt, an unterschiedlichen Enden in einer Längsrichtung des Trägermediums ausgebildet sein.

Das holographische Element 14, das sich im Transceiverbereich 16 befindet, kann mittels eines Holographieverfahrens belichtet worden sein, wodurch sich eine vorgegebene Transceiverablenkstruktur 20 bilden kann, die insbesondere als ein holographisches Volumengitter ausgebildet sein kann. Das bedeutet, dass die Transceiverablenkstruktur 20 eine Gitterstruktur aufweist, die Licht mit einer vorgegebenen Wellenlänge in einen vorbestimmten Winkel beugen kann.

Ähnlich dazu kann das holographische Element 14 in dem Messbereich 18 mit einem Holographieverfahren belichtet worden sein, wodurch sich eine Messablenkstruktur 22 bilden kann, die in diesem Beispiel auch als holographisches Volumengitter ausgebildet ist.

An einer Oberfläche des Trägermediums im Transceiverbereich 16 kann eine Transceivervorrichtung 24 vorgesehen sein, die beispielsweise mittig auf einer der Glasplatten des Trägermediums im Transceiverbereich angeordnet ist. Die Transceivervorrichtung 24 kann eine erste Lichtquelle 26 umfassen, die dazu ausgebildet ist, Licht mit einer ersten Wellenlänge auf den Transceiverbereich 16 abzustrahlen. Insbesondere kann die erste Lichtquelle 26 eine Leuchtdiode oder eine Laserdiode aufweisen, die Licht mit einer ersten Wellenlänge abstrahlt, wobei das Licht mit der ersten Wellenlänge in einem Bereich von 600 Nanometern bis 800 Nanometern liegen kann, insbesondere bei 660 Nanometern. Des Weiteren kann in der Transceivervorrichtung 24 eine Detektionsvorrichtung 28 vorgesehen sein, die lichtempfindlich ist, und zumindest ein erstes Intensitätssignal der ersten Wellenlänge bestimmen kann. Beispielsweise kann die Detektionsvorrichtung 28 eine Fotodiode aufweisen, die bei Belichtung in Abhängigkeit von der Intensität des auftreffenden Lichts einen Fotostrom verändern kann, wodurch ein erstes Intensitätssignal bestimmbar ist.

Im Folgenden soll eine Betriebsweise der in Fig. 1 gezeigten Spektrometrievorrichtung 10 anhand eines Beispiels erläutert werden. In dem in Fig. 1 gezeigten Ausführungsbeispiel kann es vorgesehen sein, dass als medizinischer Kennwert eine Pulsfrequenz bestimmt werden soll. Hierzu kann die erste Lichtquelle 26 der Transceivervorrichtung 24 beispielsweise Licht mit einer Wellenlänge von 660 Nanometern in den Transceiverbereich 16 des Trägermediums 12 abstrahlen, was als gestrichelte Linie in der Figur angedeutet ist. Das Licht der ersten Wellenlänge kann dann an dem holographischen Volumengitter der Transceiverablenkstruktur 20 derart gebeugt werden, dass das Licht in das Trägermedium 12 in Richtung des Messbereichs eingekoppelt wird und mittels interner Reflexion, das heißt Totalreflexion, zu dem Messbereichs 18 übermittelt wird (gestrichelte Linie).

In dem Messbereich 18 kann dann das holographische Volumengitter der Messablenkstruktur 22 das Licht in Richtung einer Oberfläche des Trägermediums beugen, wobei auf die Oberfläche des Trägermediums beispielsweise ein Finger 30 als zu messendes biologisches Gewebe aufliegt. Das auf den Finger abgestrahlte Licht, insbesondere das Licht mit der Wellenlänge von 660 Nanometern, kann in das biologische Gewebe eintreten und beispielsweise an Blut innerhalb des Fingers 30 gestreut und somit reflektiert werden. Das reflektierte Licht, das als gepunktete Linie angedeutet ist, kann dann zurück in den Messbereich 18 eintreten und von der Messablenkstruktur 22 zurück in das Trägermedium 12 eingekoppelt werden, wo es dann zu dem Transceiverbereich 16 zurückgeleitet wird und von der Transceiverablenkstruktur 20 auf die Transceivervorrichtung 24 ausgekoppelt werden kann. Die Detektionsvorrichtung 28 kann dann das von dem Finger 30 reflektierte Licht der ersten Wellenlänge von 660 Nanometern als ein Intensitätssignal aufnehmen, wobei sich das Intensitätssignal entsprechend einem Puls des durch den Finger 30 strömenden Blutes periodisch ändern kann.

Aus dem Intensitätssignal kann dann eine Auswerteeinrichtung 32 ein Pulsfrequenzsignal bestimmen, beispielsweise durch eine Fourier-Analyse des Intensitätssignals.

In Fig. 2 ist eine Spektrometrievorrichtung 10 gemäß einer beispielhaften Ausführungsform dargestellt. Vom generellen Aufbau ist die zweite Spektrometrievorrichtung 10 gleich zu der Spektrometrievorrichtung gemäß Figur 1 ausgebildet, das heißt mit dem Trägermedium 12, das in einer Sandwichbauweise mit zwei Glasscheiben als Deckschichten und dem holographisch-optischen Element 14 dazwischen ausgebildet ist. Des Weiteren weist die Spektrometrievorrichtung 10 der Ausführungsform die Transceivervorrichtung 24 und den Transceiverbereich 16 und den Messbereich 18 auf.

Zusätzlich zu der Spektrometrievorrichtung 10 gemäß Figur 1 umfasst die Transceivervorrichtung 24 neben der ersten Lichtquelle 26 eine zweite Lichtquelle 34, die als Leuchtdiode oder Laserdiode ausgebildet sein kann und vorzugsweise eine zweite Wellenlänge in einem Bereich von 850 Nanometern bis 1000 Nanometern, insbesondere 940 Nanometern, abstrahlen kann.

In der Ausführungsform kann die vorgegebene Transceiverablenkstruktur 20 und die vorgegebene Messablenkstruktur 22 ein holographisches Volumengitter aufweisen, das in dieser Ausführungsform als eine Multiplex-Beugungsstruktur ausgebildet ist. Mit der Multiplex-Beugungsstruktur, ist gemeint, dass das holographisch-optische Element 14 bei der Herstellung in dem Transceiverbereich beziehungsweise Messbereich derart belichtet worden ist, dass zwei Gitterstrukturen verschränkt ineinander erzeugt werden können, sodass für die jeweilige verwendete Wellenlänge ein Braggwinkel entstehen kann, der nur die Wellenlängen der ersten und zweiten Lichtquelle 26, 34 in dem vorbestimmten Winkel beugt, sodass nur dieses Licht in das Trägermedium 12 eingekoppelt und ausgekoppelt werden kann. Die Multiplex-Beugungsstruktur kann auch aufeinanderfolgende Volumengitter umfassen, wobei dafür jeweiligen aufeinanderfolgenden Volumengitter für eine vorgegebene Wellenlänge ausgebildet sind. Hierdurch ergibt sich der Vorteil, dass nur Licht mit der jeweiligen vorgegebenen Wellenlänge in das Trägermedium 12 eingekoppelt wird und Umgebungslicht, das nicht zur Messung beiträgt, herausgefiltert werden kann wodurch ein Signalrauschen an der Detektionsvorrichtung 28 verringert werden kann.

Die Detektionsvorrichtung 28 kann in dieser Ausführungsform dazu ausgebildet sein, zwischen der ersten Wellenlänge und der zweiten Wellenlänge zu unterscheiden. Dies kann beispielsweise durch Strahlteiler oder Farbfilter im Detektor erreicht werden. Mittels der Diskriminierung der Wellenlängen innerhalb der Detektionsvorrichtung 28 kann dann ein erstes Intensitätssignal der ersten Wellenlänge und ein zweites Intensitätssignal der zweiten Wellenlänge bestimmt werden, woraus Reflexions- oder Absorptionscharakteristiken des biologischen Gewebes, insbesondere des Blutes, in unterschiedlichen Spektralbereichen bestimmt werden können. Daraus kann dann mittels bekannter Verfahren eine Blutsauerstoffsättigung als medizinischer Kennwert von der Auswerteeinrichtung 32 bestimmt werden.

Um die Detektionsvorrichtung 28 vor Außenlicht, das die Messung stören kann, zu schützen, kann zusätzlich ein Gehäuse 36 vorgesehen sein, das aus einem lichtundurchlässigen, das heißt lichtabsorbierenden Material hergestellt ist. Beispielsweise kann das Gehäuse 36 ein mattes Plastikgehäuse oder ein Gehäuse aus Metall sein. Das Gehäuse 36 ist nicht auf die zweite Ausführungsform beschränkt, sondern kann auch für die erste Ausführungsform vorgesehen sein.

Mittels der Spektrometrievorrichtung 10 gemäß Figuren 1 und 2 kann erreicht werden, dass der Messbereich 18 entfernt vom Transceiverbereich 16 angeordnet werden kann, wodurch eine flachere Bauweise entstehen kann, was Platz einspart und einen besseren Zugang zu dem Messbereich ermöglicht.

Insgesamt zeigen die Beispiele, wie durch die Erfindung eine Pulsoximetrie über ein holographisch-optisches Element erreicht werden kann.

## Patentansprüche

1. Spektrometrievorrichtung (10) zur nichtinvasiven Messung von zumindest einem medizinischen Kennwert an einem biologischen Gewebe (30), umfassend
- ein Trägermedium (12), welches als Lichtleiter zum Übertragen vom eingekoppeltem Licht durch interne Reflexion ausgebildet ist, an welchem ein Transceiverbereich (16) und ein Messbereich (18), der entlang einer Längserstreckungsrichtung des Trägermediums bezüglich des Transceiverbereichs versetzt angeordnet und zum Auflegen des biologischen Gewebes vorgesehen ist, bereitgestellt ist;
- eine Transceivervorrichtung (24) mit zumindest einer ersten Lichtquelle (26) und einer Detektionsvorrichtung (28); wobei
- die erste Lichtquelle (26) dazu ausgebildet und angeordnet ist, Licht mit einer ersten Wellenlänge auf den Transceiverbereich (16) abzustrahlen; wobei
- der Transceiverbereich (16) mit einer vorgegebenen Transceiverablenkstruktur (20) ausgebildet ist, die dazu ausgelegt ist, zumindest das von der ersten Lichtquelle (26) abgestrahlte Licht der ersten Wellenlänge in das Trägermedium (12) in Richtung des Messbereichs einzukoppeln;
- der Messbereich (18) mit einer vorgegebenen Messablenkstruktur (22) ausgebildet ist, die dazu ausgelegt ist, zumindest in das Trägermedium (12) in Richtung des Messbereichs eingekoppeltes Licht aus dem Trägermedium (12) auszukoppeln, sodass bei aufgelegtem biologischen Gewebe (30) auf eine Messoberfläche des Trägermediums im Messbereich das biologische Gewebe (30) das ausgekoppelte Licht außerhalb des Trägermediums reflektiert, und wobei die vorgegebene Messablenkstruktur (22) ferner dazu ausgebildet ist, das von außerhalb des Trägermediums reflektierte Licht zu empfangen und derart abzulenken, dass es zurück in das Trägermedium (12) in Richtung des Transceiverbereichs (16) eingekoppelt wird; wobei
- die vorgegebene Transceiverablenkstruktur (20) des Transceiverbereichs (16) ferner dazu ausgebildet ist, dass das Licht, das von dem Messbereich (18) zurück in das Trägermedium (12) in Richtung des Transceiverbereichs (16) eingekoppelt wird, aus dem Trägermedium (12) auf die Detektionsvorrichtung (28) der Transceivervorrichtung (24) auszukoppeln; wobei
- die Detektionsvorrichtung (28) dazu ausgebildet ist, ein erstes Intensitätssignal der ersten Wellenlänge zu bestimmen; und umfassend
- einer Auswerteeinrichtung (32), die dazu ausgebildet ist, mittels eines zeitlichen Verlaufs des ersten Intensitätssignals ein Pulsfrequenzsignal und/oder Pulsverlaufsignal als medizinischen Kennwert zu bestimmen;
- wobei die Transceivervorrichtung (24) ferner eine zweite Lichtquelle (34) umfasst, die dazu ausgebildet und angeordnet ist, Licht mit einer zweiten, von der ersten Wellenlänge verschiedenen, Wellenlänge auf den Transceiverbereich (16) abzustrahlen; wobei
- die vorgegebene Transceiverablenkstruktur (20) des Transceiverbereichs ferner dazu ausgebildet ist, das von der zweiten Lichtquelle (34) abgestrahlte Licht der zweiten Wellenlänge in das Trägermedium (12) in Richtung des Messbereichs einzukoppeln;
- wobei die vorgegebene Messablenkstruktur (22) des Messbereichs (18) ferner dazu ausgelegt ist, das in Richtung des Messbereichs eingekoppelte Licht mit der zweiten Wellenlänge aus dem Trägermedium (12) auf das biologische Gewebe auszukoppeln und nach Reflexion an dem biologischen Gewebe zurück in das Trägermedium (12) in Richtung der Transceiverablenkstruktur (20) einzukoppeln;
- wobei die Transceiverablenkstruktur (20) ferner dazu ausgebildet ist, das reflektierte und zurück in das Trägermedium (12) eingekoppelte Licht mit der zweiten Wellenlänge aus dem Trägermedium (12) auf die Detektionsvorrichtung (28) der Transceivervorrichtung (24) auszukoppeln; und wobei
- die Detektionsvorrichtung (28) ferner dazu ausgebildet ist, ein zweites Intensitätssignal der zweiten Wellenlänge zu bestimmen; wobei
- die Auswerteeinrichtung (32) ferner dazu ausgebildet ist, aus dem ersten Intensitätssignal und dem zweiten Intensitätssignal eine Blutsauerstoffsättigung als medizinischen Kennwert zu bestimmen;
**dadurch gekennzeichnet, dass**
- die vorgegebene Transceiverablenkstruktur (20) und die vorgegebene Messablenkstruktur (22) zumindest ein optisches Gitter aufweisen; und
- das zumindest eine optische Gitter ein holographisches Oberflächengitter oder ein holographisches Volumengitter ist, das eine Multiplex-Beugungsstruktur für zumindest die erste und die zweite Wellenlänge aufweist.

2. Spektrometrievorrichtung (10) nach Anspruch 1, wobei die erste Wellenlänge in einem Bereich von 600 nm bis 800 nm liegt, insbesondere bei 660 nm.

3. Spektrometrievorrichtung (10) nach einem der vorhergehenden Ansprüche, wobei die zweite Wellenlänge in einem Bereich von 850 nm bis 1000 nm liegt, insbesondere bei 940 nm.

4. Spektrometrievorrichtung (10) nach einem der vorhergehenden Ansprüche, wobei die Detektionsvorrichtung (28) dazu ausgebildet ist, zwischen der ersten Wellenlänge und der zweiten Wellenlänge zu unterscheiden.

5. Spektrometrievorrichtung (10) nach einem der vorhergehenden Ansprüche, wobei der Transceiverbereich (16) und die Transceivervorrichtung (24) in einem von Außenlicht geschützten Gehäuse (36) untergebracht sind.

6. Spektrometrievorrichtung (10) nach einem der vorhergehenden Ansprüche, wobei das zumindest eine optische Gitter ausgebildet ist, in Abhängigkeit von einer Gitterkonstante nur Licht, insbesondere nur einen Teilbereich des sichtbaren Lichts, eines vorgegebenen Wellenlängenbereichs in einem vorbestimmten Winkel zu beugen.

7. Spektrometrievorrichtung (10) nach einem der vorhergehenden Ansprüche, wobei der Messbereich (18) und der Transceiverbereich (16) direkt in das Trägermedium (12), insbesondere in eine Oberflächenstruktur des Trägermediums, eingearbeitet sind oder
das Trägermedium (12) als separates Element zu dem Messbereich (18) und dem Transceiverbereich (16) ausgebildet ist.

## Claims

1. A spectrometry device (10) for non-invasive measurement of at least one medical characteristic value on a biological tissue (30), comprising
- a carrier medium (12), which is formed as a light guide for transferring coupled-in light by internal reflection, on which a transceiver area (16) and a measurement area (18), which is arranged offset with respect to the transceiver area along a longitudinal extension direction of the carrier medium and provided for applying the biological tissue, are provided;
- a transceiver device (24) with at least one first light source (26) and a detection device (28); wherein
- the first light source (26) is formed and arranged to radiate light with a first wavelength onto the transceiver area (16); wherein
- the transceiver area (16) is formed with a preset transceiver deflection structure (20), which is adapted to couple at least the light of the first wavelength radiated by the first light source (26) into the carrier medium (12) towards the measurement area;
- the measurement area (18) is formed with a preset measurement deflection structure (22), which is adapted to couple at least light coupled into the carrier medium (12) towards the measurement area out of the carrier medium (12), such that with applied biological tissue (30) on a measurement surface of the carrier medium in the measurement area, the biological tissue (30) reflects the coupled-out light outside of the carrier medium, and wherein the preset measurement deflection structure (22) is further formed to receive the light reflected from outside of the carrier medium and to deflect it such that it is coupled back into the carrier medium (12) towards the transceiver area (16); wherein
- the preset transceiver deflection structure (20) of the transceiver area (16) is further formed to couple the light, which is coupled from the measurement area (18) back into the carrier medium (12) towards the transceiver area (16), out of the carrier medium (12) onto the detection device (28) of the transceiver device (24); wherein
- the detection device (28) is formed to determine a first intensity signal of the first wavelength; and comprising
- an evaluation device (32), which is formed to determine a pulse frequency signal and/or pulse course signal as the medical characteristic value by means of a temporal course of the first intensity signal;
- wherein the transceiver device (24) further includes a second light source (34), which is formed and arranged to radiate light with a second wavelength different from the first wavelength onto the transceiver area (16); wherein
- the preset transceiver deflection structure (20) of the transceiver area is further formed to couple the light of the second wavelength radiated by the second light source (34) into the carrier medium (12) towards the measurement area;
- wherein the preset measurement deflection structure (22) of the measurement area (18) is further adapted to couple the light with the second wavelength coupled-in towards the measurement area out of the carrier medium (12) onto the biological tissue and to couple it back into the carrier medium (12) towards the transceiver deflection structure (20) after reflection on the biological tissue;
- wherein the transceiver deflection structure (20) is further formed to couple the reflected light with the second wavelength coupled back into the carrier medium (12) out of the carrier medium (12) onto the detection device (28) of the transceiver device (24); and wherein
- the detection device (28) is further formed to determine a second intensity signal of the second wavelength; wherein
- the evaluation device (32) is further formed to determine a blood oxygen saturation as the medical characteristic value from the first intensity signal and the second intensity signal;
**characterized in that**
- the preset transceiver deflection structure (20) and the preset measurement deflection structure (22) comprise at least one optical grating; and
- the at least one optical grating is a holographic surface grating or a holographic volume grating, which comprises a multiplex diffraction structure for at least the first and the second wavelength.

2. The spectrometry device (10) according to claim 1, wherein the first wavelength is in a range from 600 nm to 800 nm, in particular at 660 nm.

3. The spectrometry device (10) according to any one of the preceding claims, wherein the second wavelength is in a range from 850 nm to 1000 nm, in particular at 940 nm.

4. The spectrometry device (10) according to any one of the preceding claims, wherein the detection device (28) is formed to distinguish between the first wavelength and the second wavelength.

5. The spectrometry device (10) according to any one of the preceding claims, wherein the transceiver area (16) and the transceiver device (24) are accommodated in a housing (36) protected from external light.

6. The spectrometry device (10) according to any one of the preceding claims, wherein the at least one optical grating is formed to diffract only light, in particular only a partial range of the visible light, of a preset wavelength range at a predetermined angle depending on a grating constant.

7. The spectrometry device (10) according to any one of the preceding claims, wherein the measurement area (18) and the transceiver area (16) are directly incorporated into the carrier medium (12), in particular into a surface structure of the carrier medium, or the carrier medium (12) is formed as a separate element to the measurement area (18) and the transceiver area (16).

## Revendications

1. Dispositif de spectrométrie (10) pour une mesure non invasive d'au moins une valeur caractéristique médicale sur un tissu biologique (30), comprenant :
- un support (12) formé comme un conduit de lumière pour transmettre, par réflexion interne, de la lumière couplée, support sur lequel sont fournies une région d'émetteur-récepteur (16) et une région de mesure (18) agencée de manière décalée le long d'une direction d'extension longitudinale du support par rapport à la région d'émetteur-récepteur et destinée à une application du tissu biologique ;
- un dispositif émetteur-récepteur (24) avec au moins une première source de lumière (26) et un dispositif de détection (28) ;
- la première source de lumière (26) étant configurée et agencée pour émettre de la lumière à une première longueur d'onde sur la région d'émetteur-récepteur (16) ;
- la région d'émetteur-récepteur (16) étant configurée avec une structure de déviation d'émetteur-récepteur (20) prédéfinie qui est conçue pour coupler au moins la lumière à la première longueur d'onde émise à partir de la première source de lumière (26), dans le support (12) en direction de la région de mesure ;
- la région de mesure (18) étant configurée avec une structure de déviation de mesure (22) prédéfinie qui est conçue pour découpler au moins la lumière couplée dans le support (12) en direction de la région de mesure à l'extérieur du support (12), de telle sorte que lorsque le tissu biologique (30) est appliqué sur une surface de mesure du support dans la région de mesure, le tissu biologique (30) réfléchit la lumière découplée à l'extérieur du support, et la structure de déviation de mesure (22) prédéfinie étant en outre configurée pour recevoir la lumière réfléchie depuis l'extérieur du support et pour la dévier de telle sorte qu'elle est de nouveau couplée dans le support (12) en direction de la région d'émetteur-récepteur (16) ;
- la structure de déviation d'émetteur-récepteur (20) prédéfinie de la région d'émetteur-récepteur (16) étant en outre configurée de telle sorte que la lumière, qui est de nouveau couplée à partir de la région de mesure (18) dans le support (12) en direction de la région d'émetteur-récepteur (16), est découplée du support (12) sur le dispositif de détection (18) du dispositif émetteur-récepteur (24) ;
- le dispositif de détection (28) étant configuré pour déterminer un premier signal d'intensité de la première longueur d'onde ; et comprenant
- un dispositif d'évaluation (32) configuré pour déterminer, au moyen d'une courbe temporelle du premier signal d'intensité, un signal de fréquence d'impulsion et/ou un signal de courbe d'impulsion en tant que valeur caractéristique médicale ;
- le dispositif émetteur-récepteur (24) comprenant en outre une seconde source de lumière (34) configurée et agencée pour émettre de la lumière à une seconde longueur d'onde, différente de la première longueur d'onde, sur la région d'émetteur-récepteur (16) ;
- la structure de déviation d'émetteur-récepteur (20) prédéfinie de la région d'émetteur-récepteur étant en outre configurée pour coupler la lumière émise à partir de la seconde source de lumière (34) à la seconde longueur d'onde dans le support (12) en direction de la région de mesure ;
- la structure de déviation de mesure (22) prédéfinie de la région de mesure (18) étant en outre conçue pour découpler sur le tissu biologique la lumière à la seconde longueur d'onde couplée en direction de la région de mesure et provenant du support (12) et pour la coupler de nouveau dans le support (12) en direction de la structure de déviation d'émetteur-récepteur (20) après réflexion sur le tissu biologique ;
- la structure de déviation d'émetteur-récepteur (20) étant en outre configurée pour découpler la lumière réfléchie à la seconde longueur d'onde et de nouveau dans le support (12), depuis l'extérieur du support (12) sur le dispositif de détection (28) du dispositif émetteur-récepteur (24) ; et
- le dispositif de détection (28) étant en outre configuré pour déterminer un second signal d'intensité à la seconde longueur d'onde ;
- le dispositif d'évaluation (32) étant en outre configuré pour déterminer, à partir du premier signal d'intensité et du second signal d'intensité, une saturation en oxygène du sang en tant que valeur caractéristique médicale ; **caractérisé en ce que**
- la structure de déviation d'émetteur-récepteur (20) prédéfinie et la structure de déviation de mesure (22) prédéfinie comportent au moins un réseau optique ; et
- le au moins un réseau optique est un réseau holographique surfacique ou un réseau holographique volumique qui comporte une structure de diffraction multiplexée pour au moins les première et seconde longueurs d'onde.

2. Dispositif de spectrométrie (10) selon la revendication 1, dans lequel la première longueur d'onde est dans une plage de 600 nm à 800 nm et est en particulier égale à 660 nm.

3. Dispositif de spectrométrie (10) selon l'une des revendications précédentes, dans lequel la seconde longueur d'onde est dans une plage de 850 nm à 1 000 nm et est en particulier égale à 940 nm.

4. Dispositif de spectrométrie (10) selon l'une des revendications précédentes, dans lequel le dispositif de détection (28) est configuré pour différencier la première longueur d'onde de la seconde longueur d'onde.

5. Dispositif de spectrométrie (10) selon l'une des revendications précédentes, dans lequel la région d'émetteur-récepteur (16) et le dispositif émetteur-récepteur (24) sont placés dans un boîtier (36) protégé de la lumière extérieure.

6. Dispositif de spectrométrie (10) selon l'une des revendications précédentes, dans lequel le au moins un réseau optique est configuré pour diffracter uniquement la lumière, en particulier uniquement une région partielle de la lumière visible, dans les limites d'une gamme de longueurs d'onde prédéfinie à un angle prédéfini, en fonction d'une constante de réseau.

7. Dispositif de spectrométrie (10) selon l'une des revendications précédentes, dans lequel la région de mesure (18) et la région d'émetteur-récepteur (16) sont directement incorporées dans le support (12), en particulier dans une structure de surface du support ou
le support (12) est formé comme un élément séparé de la région de mesure (18) et de la région d'émetteur-récepteur (16).
